# EUROPEAN PATENT SPECIFICATION

(11) **EP 1 404 636 B1**
(45) Date of publication and mention of the grant of the patent: **10.12.2008**
(21) Application number: 02780880.7
(22) Date of filing: 13.06.2002
(51) Int. Cl.: C07C 29/124, C07C 31/04, C07C 41/01, C07C 43/04, C07C 17/10, C07C 19/075

(54) **INTEGRATED PROCESS FOR SYNTHESIZING ALCOHOLS AND ETHERS FROM ALKANES**
INTEGRIERTES VERFAHREN ZUR SYNTHESE VON ALKOHOLEN UND ETHERN AUS ALKANEN
PROCEDE INTEGRE DE SYNTHESE D'ALCOOLS ET D'ETHERS A PARTIR D'ALCANES

(30) Priority: 20.06.2001 US 886078; 11.09.2001 US 951570
(43) Date of publication of application: 07.04.2004
(60) Divisional application: 04075889.8
(73) Proprietor: GRT, Inc., The Woodlands, TX 77381 (US); The Regents of the University of California, Oakland, CA 94607 (US)
(72) Inventor: ZHOU, Xiao, Ping, Changsha Hunana 410 082 (CN); LORKOVIC, Ivan, Marc, Santa Barbara, CA 93101 (US); STUCKY, Galen, D., Goleta, CA 93117 (US); FORD, Peter, C., Santa Barbara, CA 93110 (US); SHERMAN, Jeffrey, H., Sebastian, FL 32958 (US); GROSSO, Philip, Auburn, CA 95602 (US)
(74) Representative: Jackson, Richard Eric
(86) International application number: PCT/US2002/018775
(87) International publication number: WO 2003/000635

(56) References cited:
- US-A- 4 301 253
- US-A- 4 333 852
- US-A- 5 243 098
- US-A- 5 998 679
- US-B1- 6 403 840
- OLAH, GEORGE A. ET AL: "Antimony pentafluoride/graphite catalyzed oxidative conversion of methyl halides with copper oxides (or copper/oxygen) to dimethyl ether" JOURNAL OF ORGANIC CHEMISTRY , 55(14), 4289-93 CODEN: JOCEAH; ISSN: 0022-3263, 1990, XP002283993

## Description

### TECHNICAL FIELD

This invention relates generally to the synthesis of alcohols and ethers from alkanes, and more particularly to a method of manufacturing methanol and dimethyl ether from methane.

### BACKGROUND OF THE INVENTION

Methane has previously been converted to methanol by the halogenation of methane followed by hydrolysis of the methyl halide to form methanol. For example, gaseous chlorine has been used to chlorinate methane to form chlorinated methane, principally methyl chloride, together with other chlorides, i.e., dichloromethane, trichloromethane and carbon tetrachloride. Alternatively, methane has been subjected to oxychlorination with oxygen and hydrochloric acid to form the foregoing compounds. The chlorinated methanes produced are hydrolyzed in the vapor phase to produce methanol, formaldehyde, formic acid and by-products, including carbon dioxide and hydrochloric acid, depending on the chlorination selectivity. Hydrochloric acid is produced or used in the halogenation of methane by either method and must be recovered, dehydrated by azeotropic distillation and recycled. Corrosion and other problems involved with the handling of chlorine and hydrochloric acid are substantial.

U.S. Patent No. 3,172,915 granted to Borkowski, et al. is directed to a process for converting methane to methanol. Borkowski discloses the chlorination of methane using ferric chloride at high temperatures to produce chloromethanes and hydrogen chloride. The process requires temperatures in the range of 220-800º C., more preferably 250-450º C., and long residence times, e.g., more than one hour. Further, the process is hindered by the production of a mixture of chlorination products, e.g., chloromethane, dichloromethane, trichloromethane and carbon tetrachloride, which must be separated before hydrolysis to methanol. Other disadvantages result from the energy required to dry the ferric chloride and from the corrosion and handling problems inherent with hydrochloric acid.

U.S. Patent No. 5,243,098 granted to Miller discloses another method for converting methane to methanol. In the Miller process the reaction of methane with cupric chloride produces chloromethane and hydrochloric acid. These intermediates are then reacted with steam and a catalyst containing magnesium oxide to produce methanol and magnesium chloride. Magnesium oxide is regenerated by treatment of the magnesium chloride by-product with air or oxygen. Cupric chloride is regenerated by treatment of the cuprous chloride by-product with air and hydrochloric acid. While these reactions proceed at favorable rates, attrition of the solid reactants, i.e., cupric and magnesium oxide, is significant. Special filters and processes are required to recover and regenerate the reactants in the required particle size. Miller also suggests cupric bromide and magnesium zeolite as alternative reactants. Because of the attrition of the reactants, difficulties associated with the handling of solids, and the special filters and processes required to regenerate the reactants, the Miller process has proven unsatisfactory. U.S. Pat. No. 5,334,777, also granted to Miller, discloses a nearly identical process for converting ethene to ethylene glycol.

U.S. Patent No. 5,998,679 granted to Jorge Miller, discloses a process for converting alkanes and alkenes to the corresponding lower alkanols and diols. In the method of the invention, a gaseous halogen (bromine) is produced by decomposing a metal halide in a liquid having a melting point below and a boiling point above the decomposition temperature of the metal halide. The preferred liquid is molten hydrated ferric chloride maintained at a temperature between about 37-280º C. The lower alkane or alkene is halogenated in a gas phase reaction with the halogen. The resulting alkyl halide or alkyl dihalide is contacted with a metal hydroxide, preferably an aqueous solution of ferric hydroxide, to regenerate the metal halide and produce the corresponding lower alkanol or diol. Problems with this process include low monohalogenation selectivity, and corrosiveness of the hydrated ferric halides, which may present a containment problem if the process is run at 280º C, where high pressures of steam are required to maintain ferric halide hydration. Finally, the process produces a great deal of water and HCl or HBr, all of which are difficult to separate on a large scale from the desired product methanol.

Published international patent application WO 00/07718, naming Giuseppe Bellussi, Carlo Perego, and Laura Zanibelli as inventors, discloses a method for directly converting methane and oxygen to methanol over a metal halide/metal oxide catalyst. This is not a catalyst in the true sense of the word, however, because the reaction involves transfer of halide from a metal halide via reaction with methane to a different metal oxide producing the metal halide and methanol downstream. Eventually the halide is leached and the catalyst loses activity.

Olah et al. (George A. Olah, et al. J. Am. Chem. Soc. 1985, 107, 7097-7105) discloses a method for converting methane to methanol via methyl halides (CH₃Br and CH₃Cl), which are then hydrolyzed to prepare methanol. In the process, CH₃Br and CH₃Cl are hydrolyzed over catalysts with excess steam generating a methanol, water, and HCl or HBr mixture. The separation of methanol (about 2% by mole) from HCl or HBr and water on an industry scale (2000 tons per day) requires an enormous amount of energy and generates a great deal of aqueous HCl or HBr waste. Aqueous HCl and HBr are very corrosive as well.

### SUMMARY OF THE INVENTION

The present invention comprises a process wherein bromine or a bromine-containing compound is used as an intermediate to convert alkanes to alcohols, ethers, or olefins by reaction with oxygen or air. While the process can be used to convert a variety of alkanes, including methane, ethane, propane, butane, isobutane, pentanes, hexanes, cyclohexane, etc. to their respective alcohols, ethers, or olefins, the conversion of methane to methanol and dimethyl ether is illustrative.

Methane reacts with bromine over a catalyst to form CH₃Br and HBr. CH₃Br and HBr react with a metal oxide to form a variable mixture of dimethyl ether (DME), water and methanol, and the metal bromide. The metal oxide and molecular bromine are regenerated by reaction of the metal bromide with air and/or oxygen. The regenerated bromine is recycled to react with methane while the regenerated metal oxide is used to convert more methyl bromide to methanol and DME, completing the reaction cycle.

The process can be easily carried out in a riser reactor. Compared to the current industrial two-step process, in which methane and steam are first converted to CO and H₂ at 800º C followed by conversion to methanol over a Zn-Cu-Al-O catalyst at approximately 70-150 atmospheres, the process of the present invention operates at roughly atmospheric pressure and relatively low temperatures, thereby providing a safe and efficient process for methanol production.

The present invention operates with solid/gas mixtures at atmospheric pressure. In the process, the hydrogen halide is gaseous, and therefore not as corrosive as when aqueous at high temperatures. The reaction of Br₂ with an alkane can reach more than 90% selectivity with high conversion to alkane-monobromide. The main side products, alkane dibromides such as CH₂Br₂, can be converted back to the monobromides by reaction with an alkane over another catalyst. Very few by-products are produced.

In the process most of the Br atoms are trapped in the solid state, making the system less corrosive. Another advantage is that DME and alcohol (CH₃OH) are not produced as a mixture with excess water. By controlling reaction conditions, almost pure DME and/or methanol is obtained directly so that it is not necessary to separate CH₃OH from water. Finally, in the present process, methane and oxygen do not come into direct contact, resulting in improved safety.

### BRIEF DESCRIPTION OF THE DRAWING

A more complete understanding of the present invention may be had by reference to the following Detailed Description when taken in connection with the accompanying Drawing, wherein:

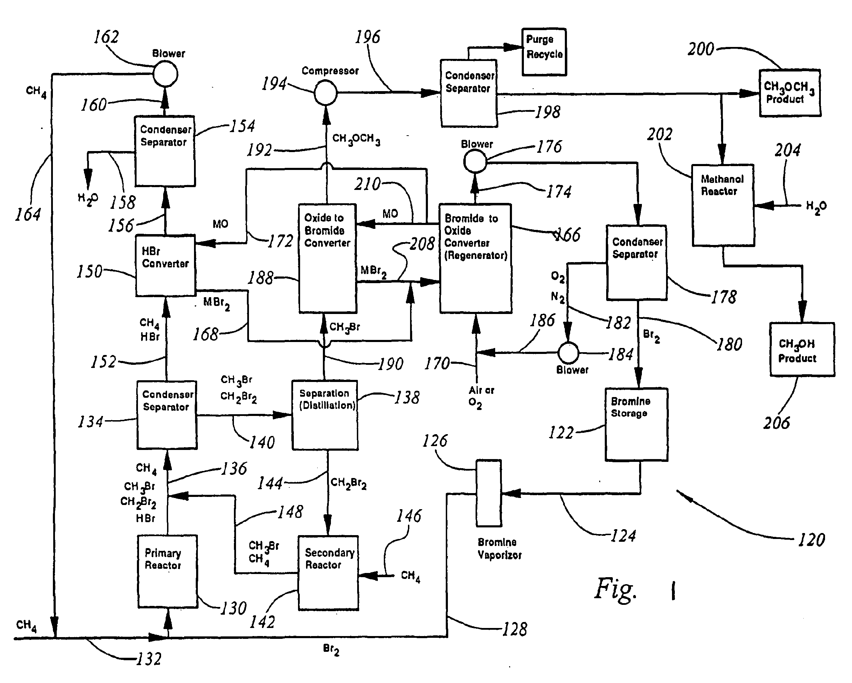

FIGURE 1 is a schematic illustration of a method of and apparatus for synthesizing ethers and/or alcohols from alkanes comprising an embodiment of the invention.

### DETAILED DESCRIPTION

Alkanes (methane, ethane, propane, butane, isobutane, pentanes, hexanes, cyclohexane, etc.) react with molecular bromine over a metal oxide catalyst selected from all possible metal oxide compounds and mixtures thereof to form alkylbromides. For CH₄ (although the process may be applied to higher alkanes as well), the process of the present invention can convert more than 50% CH₄ to CH₃Br and HBr, with selectivity higher than 90%. Most of the by-product is CH₂Br₂ (with trace amounts of CHBr₃, and CBr₄)(+ 2 HBr), which can be catalytically reconverted to CH₃Br by reacting CH₂Br₂ with CH₄.

A method and apparatus for synthesizing alcohols and ethers from alkanes using bromine comprising a first embodiment of the invention is provided. In the operation of the method and apparatus, bromine is received from a suitable source through a first line and is directed to a bromine storage container. For example, bromine is easily manufactured from bromide, which is readily available from sea water.

As is well known, bromine is a liquid at room temperature. Liquid bromine from the storage container is directed through a second line to a bromine vaporizer wherein the bromine is converted from the liquid phase to the gas phase. From the vaporizer the gaseous bromine is directed through a third line to a reactor.

Methane from a suitable source is directed to the reactor, which contains a bromination catalyst, through a fourth line. Within the reactor the methane and the gaseous bromine are mixed together and the temperature of the mixture is raised to between 20º C and 600º C, thereby converting the methane and the bromine to methyl bromide (CH₃Br) and hydrogen bromide (HBr).

From the reactor, the CH₃Br, the HBr, any unreacted methane and by products CH₂Br₂, CHBr₃, and CBr₄ are directed to a first condenser. The by products CH₂Br₂, CHBr₃, and CBr₄ now in the liquid state, are sent to a first converter with methane from the fourth line. In the first converter , methane reacts with the by products CH₂Br₂, HBr₃, and CBr₄ to form CH₃Br. The newly formed CH₃Br and any unreacted CH₂Br₂, CHBr₃, CBr₄ and/or methane are sent to the first condenser. From the first condenser, the gas phase methane, HBr, and CH₃Br are sent to a second converter. In the second converter HBr and CH₃Br react with metal oxide to form CH₃OCH₃, CH₃OH, and H₂O, which are sent to a first separator along with unreacted methane and CH₃Br. In the first separator, methanol and dimethyl ether are separated as products. CH₃Br is sent back to the second converter. Methane is sent back to the bromination reactor. In the second converter, the original metal oxide has been converted to metal bromide after reaction. The metal bromide is sent to a third converter to react with oxygen (from source) to regenerate bromine and metal oxide. The regenerated metal oxide is sent back to the second converter, while the bromine and unreacted oxygen are sent to a second condenser, then to a second separator. The liquid bromine is sent to the storage container, while oxygen is sent to the third converter.

There is also provided a method of and apparatus for synthesizing alcohols and ethers from alkanes comprising a second embodiment of the invention. Methane and bromine are directed to a heated vaporizer where they are heated to form a gas mixture. The gas mixture is passed into a bromination reactor, containing a bromination catalyst, if desired. After the reaction, the mixture is directed to a condenser. The liquid phase contains by products CH₂Br₂, CHBr₃, and CBr₄, while the gas phase contains bromomethane, HBr, and unreacted methane.

The liquid by products CH₂Br₂, CHBr₃, and CBr₄ are sent to a first converter where they react with methane to form bromomethane. After reaction the mixture is sent to the condenser, where any remaining byproducts can once again be cycled to the bromination reactor.

The gas phase mixture from condenser is passed through a second converter, where HBr reacts with metal oxide to form metal bromide and water. The metal bromide is sent to a regenerator to regenerate metal oxide. From the second converter, the water, bromomethane, and methane are separated. Methane is recycled to the first converter and the vaporizer. Bromomethane is sent to a first reactor. Water is sent to a second reactor.

In the first reactor, bromomethane reacts with metal oxide to generate dimethyl ether (DME) and metal bromide. Metal bromide is sent to the regenerator. The mixture of bromomethane and DME from the first reactor is sent to a first separator. Bromomethane from the first separator is recycled to the first reactor, while DME is obtained as a product or directed to the second reactor.

In the second reactor, DME reacts with water, e.g., from the first separator, over a suitable catalyst to form methanol.

In the regenerator, metal bromide from the second converter and the first reactor reacts with air or oxygen to regenerate metal oxide and bromine. After regeneration, metal oxide is sent to the second converter and the first reactor, while bromine is sent to the vaporizer. If air is used as the source of oxygen, nitrogen can be continuously purged from the system through a second separator.

A method and apparatus 120 for converting methane to dimethyl ether and/or methanol comprising a third embodiment of the invention is illustrated in Figure 1. Liquid bromine is stored in a bromine storage container 122 and is directed therefrom through a line 124 through a bromine vaporizer 126. From the bromine vaporizer 126 the bromine vapor passes through a line 128 to a primary reactor 130 which also receives methane from a suitable source through a line 132.

Within the primary reactor 130, the bromine and methane react over a solid catalyst, if desired, to form CH₃Br, CH₂Br₂, CHBr₃, CBr₄ and HBr. The reaction products together with any unreacted methane are directed to a condenser separator 134 through a line 136. The condenser separator 134 directs CH₃Br, CH₂Br₂, CHBr₃, and CBr₄ to a separation (distillation) apparatus 138 through a line 140. The separation (distillation) apparatus 138 directs CH₂Br₂, CHBr₃, and CBr₄ to a secondary reactor 142 through a line 144. The secondary reactor 142 also receives methane from a suitable source through a line 146.

The secondary reactor 142 converts the CH₂Br₂, CHBr₃, and CBr₄ received through the line 144 to CH₃Br. CH₃Br and excess methane are directed from the secondary reactor 142 to the line 136 through a line 148.

The condenser separator 134 also directs CH₄ and HBr to an HBr converter 150 through a line 152. The HBr converter 150 is filled with a metal oxide. Within the HBr converter 150 the metal oxide and the HBr react to form metal bromide and water. The water and the unreacted methane are directed from the HBr converter 150 to a condenser separator 154 through a line 156. The water is recovered from the condenser separator 154 through an outlet 158, while the unreacted methane is directed through a line 160 to a blower 162 and from the blower 162 through a line 164 to the line 132.

Metal bromide formed during operation of the HBr converter 150 is directed to a bromide to oxide converter (regenerator) 166 through a line 168. The bromide to oxide converter 166 receives air or oxygen through a line 170 and functions to convert the metal bromide back to metal oxide. The metal oxide is returned to the HBr converter 150 through a line 172.

Operation of the bromide to oxide converter 166 also produces bromine. Bromine and excess air or oxygen are directed through a line 174 to a blower 176, and from the blower 176 to a condenser separator 178. Operation of the condenser separator 178 produces liquid bromine which is directed to the bromine storage container 122 through a line 180. Excess air and/or oxygen is recovered from the condenser separator 178 through an outlet 182 and is directed therefrom through a blower 184 and a line 186 to the line 170.

The separation (distillation) apparatus 138 directs CH₃Br to an oxide to bromide converter 188 through a line 190. The oxide to bromide converter 188 is filled with a metal oxide, which may be the same metal oxide that is utilized in the operation of the HBr converter 150. Operation of the oxide to bromide converter 188 produces dimethyl ether, which together with unreacted bromomethane is directed through a line 192, a compressor 194, and a line 196 to a condenser separator 198. From the condenser separator 198 dimethyl ether may be recovered as a final product at an outlet 200. The bromomethane is sent back to the converter 188. Alternatively, the dimethyl ether may be directed to a methanol reactor 202, which receives water through an inlet 204. The dimethyl ether and the water react in the methyl reactor 202 to form methanol, which is recovered at an outlet 206.

Operation of the oxide to bromide converter 188 converts the metal oxide contained therein to metal bromide, which is directed to the bromide to oxide converter 166 through a line 208. Operation of the bromide to oxide converter 166 converts the metal bromide to the original metal oxide, which is returned to the oxide to bromide converter 188 through a line 210.

### EXAMPLES

### Reaction 1:

### Catalyst preparation

Nb₂O₅ (0.8000g) was mixed with 0.500ml 96(w)% H₂SO₄, then the mixture was heated at 110°C for 4 hours. The temperature increased to 500°C within 6 hours, and kept at 500°C for 4 hours. Catalyst C1 was obtained.

### Testing

### Reaction conditions:

The catalyst was tested at a methane flow of 1.5ml/minute and Br₂ flow of 0.07ml/hour. The reaction temperature was 400°C. The reaction was carried out in a microreactor system. After 6 hours on line reaction, the reaction effluent was analyzed by a GC/MS. A methane conversion of 24%(mol) with 95% CH₃Br was obtained.

Summarizing the overall process in Reaction 1:

(1) CH₄ + Br₂ > HBr + CH₃Br + CH₂Br₂ + CHBr₃ cat

### Reaction 2:

### Metal oxide preparation

### Zr solution preparation

Zr(OCH₂CH₂CH₃)₄ (70(w)% in isopropanol, 112.6 ml) was dissolved into acetic acid (275 ml) under stirring. After stirring for 10 minutes, the solution was diluted by water to make a total volume of 500 ml. A solution with a Zr concentration of 0.5M was obtained.

### Preparation of M1

Cu(NO₃)₂ (0.5M, 7.200ml) solution was added into BaBr₂ (0.5M, 0.800 ml). A clear solution was obtained. To this solution, Zr solution (0.5M) as prepared above was added under stirring. After stirring a few seconds, a gel was obtained. The gel was dried at 110°C for 4 hours, then heated to 500°C within 6 hours, and kept at 500°C for 4 hours. M1 was obtained. The metal oxide mixture was tested at a CH₃Br flow of 1.0 ml/minute at 230°C. In the first half hour, the average CH₃Br conversion is 65%, and the average dimethyl ether selectivity is 90.5%.

### Catalyst preparation

ZrO₂ (2.0000g) was mixed with H₂SO₄ (3.000 ml, 96(w)%), then the mixture was heated at 110°C for 4 hours. The temperature increased to 500°C within 6 hours, and kept at 500°C for 4 hours. Catalyst C2 was obtained.

### Preparation of M2

Cu(NO₃)₂ (0.5M, 40.000 ml) solution was added into Zr solution (0.5M, 30.000 ml as prepared above). After stirring a few seconds, a gel was obtained. The gel was dried at 110°C for 4 hours, then heated to 500•C within 6 hours, and calcined at 500•C for 4 hours. M2 was obtained.

### Testing

The catalyst C2 (2.0000g) was loaded in the first reactor (R1). A trap was loaded 2.0000g M2 in the oxide form. A second reactor (R2) was also loaded with M2 in the oxide form(0.8500g).

Reactants methane and bromine were fed into the first reactor (methane flow of 1.5 ml/minute, Br₂ flow of 0.07 ml/hour). The reaction temperature was 390°C. After the reaction in R1 (stabilized by online reaction for more than 8 hours), the products generated in R1 were passed through the trap and HBr was removed. Following removal of HBr, a mixture of methane and CH₃Br (containing 20% mol of CH₃Br) was obtained. This gas mixture was directly fed into R2 at 220°C. In the first one hour, an average CH₃Br conversion of 91% with an average dimethyl ether selectivity of 75% was obtained.

Summarizing the overall process in Reaction 2:

(2) CH₃Br + HBr + CuO > CH₃OH + CuBr₂

Possible variations of Reaction 2:

(2a) 2 HBr + CuO > H₂O + CuBr₂ (reaction occurring in the trap)

(2b) 2 CH₃Br + CuO > CH₃OCH₃ + CuBr₂

### Reaction 3:

The solid CuBr₂/ZrO₂ is transferred from Reactor 2 to Reactor 3 and treated with O₂ at 300ºC to yield Br₂ and CuO/ZrO₂ in 100% yield and conversion. This reaction may be run at space velocity 100 h⁻¹.

Summarizing the overall process in Reaction 3:

(3) CuBr₂/ZrO₂ + ½ O₂ > Br₂ + CuO/ZrO₂

### Overall:

(A) CH₄ + ½ O₂ > CH₃OH

Possible variation:

(B) CH₄ + ½ O₂ > ½ CH₃OCH₃ + ½ H₂O

It will therefore be understood that the method of the present invention operates on a continuous or batch basis to convert alkanes to alcohols and ethers. The method of the present invention operates at relatively low temperatures and at low pressures and is therefore economical for manufacture and use. The bromine, which is utilized in the method of the present invention, is continuously recycled. The metal oxide catalyst, which is utilized in the process is continuously refreshed.

Although preferred embodiments of the invention have been illustrated in the accompanying Drawing and described in the foregoing Detailed Description, it will be understood that the invention is not limited to the embodiments disclosed but is capable of numerous rearrangements, modifications, and substitutions of parts and elements without departing from the scope of the invention.

## Claims

1. A method for synthesizing alcohols and/or ethers from alkanes comprising:
providing a quantity of an alkane selected from the group including methane, ethane, propane, butane, and isobutane;
providing a quantity of bromine;
mixing the alkane and the bromine and thereby forming an alkyl bromide and hydrogen bromide;
directing the hydrogen bromide into engagement with a metal oxide and thereby forming a metal bromide and water;
converting the metal bromide from the hydrogen bromide metal oxide reaction to form the original metal oxide;
separately directing the alkyl bromide into engagement with a metal oxide and thereby forming alcohol and/or ether and a metal bromide;
converting the metal bromide from the alkyl bromide metal oxide reaction to form the original metal oxide and bromine;
recycling the metal oxide; and
recycling the bromine.

2. The method according to claim 1 wherein the step of mixing the alkane and the bromine is carried out at an alkane to bromine molar ratio from 1:10 to 100:1.

3. The method according to claim 1 wherein the step of mixing the alkane and the bromine is carried out at a temperature of between 20°C and 600°C.

4. The method according to claim 1 wherein the step of mixing the alkane and the bromine is carried out at a pressure between 0.1 to 200 atm.

5. The method according to claim 1 wherein the step of reacting the alkane with the bromine to form the alkyl bromide and hydrogen bromide with metal oxide and the step of contacting the alkyl bromide with metal oxides are carried out continuously.

6. The method according to claim 1 wherein the step of reacting the alkane with the bromine to form the alkyl bromide and hydrogen bromide and the step of contacting the alkyl bromide with metal oxide are carried out in a batch reaction.

7. The method according to claim 1 wherein the step of converting the metal bromide to form the original metal oxide and bromine, the step of recycling the metal oxide, and the step of recycling the bromine are carried out continuously.

8. The method according to claim 1 wherein the step of converting the metal bromide to form the original metal oxide and bromine, the step of recycling the metal oxide, and the step of recycling the bromine are carried out in a batch reaction.

9. The method according to claim 1 wherein the alkyl bromide and the hydrogen bromide are separately directed into engagement with the same metal oxide.

10. The method according to claim 1 wherein the alkyl bromide and the hydrogen bromide are separately directed into engagement with different metal oxides.

11. A method according to any one of claims 1 to 8 for converting methane to methanol, wherein:
the alkane is methane;
the methyl bromide, provided by mixing the methane and the bromine, is directed into engagement with a metal oxide, thereby forming methanol and a metal bromide;
the metal bromide resulting from the methyl bromide metal oxide reaction is converted to form the original metal oxide and bromine; and
the hydrogen bromide is separately directed into engagement with a metal oxide to form water and a metal bromide.

12. The method according to claim 11 wherein the step of mixing the methane and the bromine is carried out at a methane to bromine ratio from 1:1 to 10:1 (by mole).

13. The process according to claim 11 wherein the step of mixing the methane and the bromine is carried out at a methane to bromine ratio from 1:1 to 5:1 (by mole).

14. A method according to any one of claims 1 to 8 for synthesizing ethers from alkanes, wherein:
the alkyl bromide, provided by mixing the alkane and the bromine is directed into engagement with a metal oxide, thereby forming an ether and a metal bromide;
the metal bromide from the alkyl bromide metal oxide reaction is converted to form metal oxide and bromine; and
the hydrogen bromide is separately directed into engagement with a metal oxide to form water and a metal bromide.

15. The method according to claim 14 wherein the step of mixing the alkane and the bromine is carried out at a alkane to bromine mol ratio between 1:5 to 50:1.

16. The method according to claim 14 wherein the step of mixing the alkane and the bromine is carried out at a alkane to bromine mol ratio between 1:2 to 10:1.

17. A method according to any one of claims 1 to 5 and 7 for converting methane to dimethyl ether, wherein:
the alkane is methane;
the hydrogen bromide, formed by mixing the methane and bromine, is trapped by reacting it with a metal oxide; and
the methyl bromide formed by mixing the methane and bromine is directed into engagement with a metal oxide, thereby forming dimethyl ether and a metal bromide.

18. The method according to claim 17 wherein the step of mixing the methane and the bromine is carried out at a methane to bromine mol ratio between 1:5 to 50:1.

19. The method according to claim 17 wherein the step of mixing the methane and the bromine is carried out at a methane to bromine mol ratio between 1:2 to 10:1.

20. The method according to claim 17 wherein the step of trapping the hydrogen bromide by reacting it with metal oxide produces water and metal bromide.

21. The method according to claim 20 wherein the metal oxide which reacts with the methyl bromide and the metal oxide which reacts with the hydrogen bromide are the same metal oxide.

22. The method according to claim 20 wherein the metal oxide which reacts with the methyl bromide and the metal oxides which reacts with the hydrogen bromide are different metal oxides.

## Patentansprüche

1. Verfahren zur Synthese von Alkoholen und/oder Ethern aus Alkanen, umfassend:
Bereitstellen einer Menge eines Alkans, das ausgewählt ist aus der Gruppe, die Methan, Ethan, Propan, Butan und Isobutan einschließt;
Bereitstellen einer Menge Brom;
Mischen des Alkans und des Broms und **dadurch** Bilden von einem Alkylbromid und Bromwasserstoff;
Leiten des Bromwasserstoffs in Kontakt mit einem Metalloxid und **dadurch** Bilden von einem Metallbromid und Wasser;
Umwandeln des Metallbromids aus der Bromwasserstoff-Metalloxid-Reaktion, um das ursprüngliche Metalloxid zu bilden;
getrenntes Leiten des Alkylbromids in Kontakt mit einem Metalloxid und **dadurch** Bilden von Alkohol und/oder Ether und einem Metallbromid;
Umwandeln des Metallbromids aus der Alkylbromid-Metalloxid-Reaktion, um das ursprüngliche Metalloxid und Brom zu bilden;
Rückführen des Metalloxids; und
Rückführen des Broms.

2. Verfahren nach Anspruch 1, wobei der Schritt des Mischens des Alkans und des Broms bei einem Alkan-Brom-Molverhältnis von 1:10 bis 100:1 durchgeführt wird.

3. Verfahren nach Anspruch 1, wobei der Schritt des Mischens des Alkans und des Broms bei einer Temperatur zwischen 20°C und 600°C durchgeführt wird.

4. Verfahren nach Anspruch 1, wobei der Schritt des Mischens des Alkans und des Broms bei einem Druck zwischen 0,1 und 200 atm durchgeführt wird.

5. Verfahren nach Anspruch 1, wobei der Schritt des Umsetzens des Alkans mit dem Brom, um das Alkylbromid und Bromwasserstoff zu bilden, mit Metalloxid, und der Schritt des Inkontaktbringens des Alkylbromids mit Metalloxiden kontinuierlich durchgeführt werden.

6. Verfahren nach Anspruch 1, wobei der Schritt des Umsetzens des Alkans mit dem Brom, um das Alkylbromid und Bromwasserstoff zu bilden, und der Schritt des Inkontaktbringens des Alkylbromids mit Metalloxid in einer diskontinuierlichen Reaktion durchgeführt werden.

7. Verfahren nach Anspruch 1, wobei der Schritt des Umwandelns des Metallbromids, um das ursprüngliche Metalloxid und Brom zu bilden, der Schritt des Rückführens des Metalloxids und der Schritt des Rückführens des Broms kontinuierlich durchgeführt werden.

8. Verfahren nach Anspruch 1, wobei der Schritt des Umwandelns des Metallbromids, um das ursprüngliche Metalloxid und Brom zu bilden, der Schritt des Rückführens des Metalloxids und der Schritt des Rückführens des Broms in einer diskontinuierlichen Reaktion durchgeführt werden.

9. Verfahren nach Anspruch 1, wobei das Alkylbromid und der Bromwasserstoff getrennt in Kontakt mit demselben Metalloxid geleitet werden.

10. Verfahren nach Anspruch 1, wobei das Alkylbromid und der Bromwasserstoff getrennt in Kontakt mit unterschiedlichen Metalloxiden geleitet werden.

11. Verfahren nach einem der Ansprüche 1 bis 8 zum Umwandeln von Methan in Methanol, wobei:
das Alkan Methan ist;
das Methylbromid, das durch Mischen des Methans und des Broms bereitgestellt wird, in Kontakt mit einem Metalloxid geleitet wird, wodurch Methanol und ein Metallbromid gebildet werden;
das Metallbromid, das aus der Methylbromid-Metalloxid-Reaktion stammt, umgewandelt wird, um das ursprüngliche Metalloxid und Brom zu bilden; und
der Bromwasserstoff getrennt in Kontakt mit einem Metalloxid geleitet wird, um Wasser und ein Metallbromid zu bilden.

12. Verfahren nach Anspruch 11, wobei der Schritt des Mischens des Methans und des Broms bei einem Methan-Brom-Verhältnis von 1:1 bis 10:1 (molbezogen) durchgeführt wird.

13. Verfahren nach Anspruch 11, wobei der Schritt des Mischens des Methans und des Broms bei einem Methan-Brom-Verhältnis von 1:1 bis 5:1 (molbezogen) durchgeführt wird.

14. Verfahren nach einem der Ansprüche 1 bis 8 zur Synthese von Ethern aus Alkanen, wobei:
das Alkylbromid, das durch Mischen des Alkans und des Broms bereitgestellt wird, in Kontakt mit einem Metalloxid geleitet wird, wodurch ein Ether und ein Metallbromid gebildet werden;
das Metallbromid aus der Alkylbromid-Metalloxid-Reaktion umgewandelt wird, um Metalloxid und Brom zu bilden; und
der Bromwasserstoff getrennt in Kontakt mit einem Metalloxid geleitet wird, um Wasser und ein Metallbromid zu bilden.

15. Verfahren nach Anspruch 14, wobei der Schritt des Mischens des Alkans und des Broms bei einem Alkan-Brom-Molverhältnis zwischen 1:5 bis 50:1 durchgeführt wird.

16. Verfahren nach Anspruch 14, wobei der Schritt des Mischens des Alkans und des Broms bei einem Alkan-Brom-Molverhältnis zwischen 1:2 bis 10:1 durchgeführt wird.

17. Verfahren nach einem der Ansprüche 1 bis 5 und 7 zum Umwandeln von Methan in Dimethylether, wobei:
das Alkan Methan ist;
der Bromwasserstoff, der durch Mischen des Methans und Broms gebildet wird, durch Umsetzen desselben mit einem Metalloxid eingefangen wird; und
das Methylbromid, das durch Mischen des Methans und Broms gebildet wird, in Kontakt mit einem Metalloxid geleitet wird, wodurch Dimethylether und ein Metallbromid gebildet werden.

18. Verfahren nach Anspruch 17, wobei der Schritt des Mischens des Methans und des Broms bei einem Methan-Brom-Molverhältnis zwischen 1:5 bis 50:1 durchgerührt wird.

19. Verfahren nach Anspruch 17, wobei der Schritt des Mischens des Methans und des Broms bei einem Methan-Brom-Molverhältnis zwischen 1:2 bis 10:1 durchgerührt wird.

20. Verfahren nach Anspruch 17, wobei der Schritt des Einfangens des Bromwasserstoffs durch Umsetzen desselben mit Metalloxid Wasser und Metallbromid erzeugt.

21. Verfahren nach Anspruch 20, wobei das Metalloxid, das mit dem Methylbromid reagiert, und das Metalloxid, das mit dem Bromwasserstoff reagiert, dasselbe Metalloxid sind.

22. Verfahren nach Anspruch 20, wobei das Metalloxid, das mit dem Methylbromid reagiert, und das Metalloxid, das mit dem Bromwasserstoff reagiert, unterschiedliche Metalloxide sind.

## Revendications

1. Procédé pour synthétiser des alcools et/ou des éthers à partir d'alcanes, comprenant les étapes consistant à:
partir d'une quantité d'un alcane choisi dans le groupe comprenant le méthane, l'éthane, le propane, le butane et l'isobutane;
partir d'une quantité de brome;
mélanger l'alcane et le brome et former ainsi un bromure d'alkyle et du bromure d'hydrogène;
diriger le bromure d'hydrogène dans un engagement avec un oxyde de métal et former ainsi un bromure de métal et de l'eau;
convertir le bromure de métal issu de la réaction bromure d'hydrogène oxyde de métal pour former l'oxyde de métal d'origine;
diriger séparément le bromure d'alkyle dans un engagement avec un oxyde de métal et former ainsi un alcool et/ou un éther et un bromure de métal;
convertir le bromure de métal issu de la réaction bromure d'alkyle oxyde de métal pour former l'oxyde de métal d'origine et du brome;
recycler l'oxyde de métal; et
recycler le brome.

2. Procédé selon la revendication 1, dans lequel l'étape de mélange de l'alcane et du brome est réalisée à un rapport molaire de l'alcane au brome de 1:10 à 100:1.

3. Procédé selon la revendication 1, dans lequel l'étape de mélange de l'alcane et du brome est réalisée à une température comprise entre 20°C et 600°C.

4. Procédé selon la revendication 1, dans lequel l'étape de mélange de l'alcane et du brome est réalisée à une pression comprise entre 0,1 et 200 atm.

5. Procédé selon la revendication 1, dans lequel l'étape de réaction de l'alcane avec le brome pour former le bromure d'alkyle et le bromure d'hydrogène avec l'oxyde de métal et l'étape de mise en contact du bromure d'alkyle avec l'oxyde de métal sont réalisées en continu.

6. Procédé selon la revendication 1, dans lequel l'étape de réaction de l'alcane avec le brome pour former le bromure d'alkyle et le bromure d'hydrogène et l'étape de mise en contact du bromure d'alkyle avec l'oxyde de métal sont réalisées dans une réaction discontinue.

7. Procédé selon la revendication 1, dans lequel l'étape de conversion du bromure de métal pour former l'oxyde de métal d'origine et du brome, l'étape de recyclage de l'oxyde de métal et l'étape de recyclage du brome sont réalisées en continu.

8. Procédé selon la revendication 1, dans lequel l'étape de conversion du bromure de métal pour former l'oxyde de métal d'origine et du brome, l'étape de recyclage de l'oxyde de métal et l'étape de recyclage du brome sont réalisées dans une réaction discontinue.

9. Procédé selon la revendication 1, dans lequel le bromure d'alkyle et le bromure d'hydrogène sont dirigés séparément dans un engagement avec le même oxyde de métal.

10. Procédé selon la revendication 1, dans lequel le bromure d'alkyle et le bromure d'hydrogène sont dirigés séparément dans un engagement avec des oxydes de métal différents.

11. Procédé selon l'une quelconque des revendications 1 à 8 pour convertir le méthane en méthanol, dans lequel:
l'alcane est le méthane;
le bromure de méthyle, obtenu en mélangeant le méthane et le brome, est dirigé dans un engagement avec un oxyde de métal, formant ainsi du méthanol et un bromure de métal;
le bromure de métal résultant de la réaction bromure de méthyle oxyde de métal est converti pour former l'oxyde de métal d'origine et du brome; et
le bromure d'hydrogène est dirigé séparément dans un engagement avec un oxyde de métal pour former de l'eau et un bromure de métal.

12. Procédé selon la revendication 11, dans lequel l'étape de mélange du méthane et du brome est réalisée à un rapport du méthane au brome de 1:1 à 10:1 (en mole).

13. Procédé selon la revendication 11, dans lequel l'étape de mélange du méthane et du brome est réalisée à un rapport du méthane au brome de 1:1 à 5:1 (en mole).

14. Procédé selon l'une quelconque des revendications 1 à 8 pour synthétiser des éthers à partir d'alcanes, dans lequel:
le bromure d'alkyle, obtenu en mélangeant l'alcane et le brome, est dirigé dans un engagement avec un oxyde de métal, formant ainsi un éther et un bromure de métal;
le bromure de métal issu de la réaction bromure d'alkyle oxyde de métal est converti pour former un oxyde de métal et du brome; et
le bromure d'hydrogène est dirigé séparément dans un engagement avec un oxyde de métal pour former de l'eau et un bromure de métal.

15. Procédé selon la revendication 14, dans lequel l'étape de mélange de l'alcane et du brome est réalisée à un rapport molaire de l'alcane au brome compris entre 1:5 et 50:1.

16. Procédé selon la revendication 14, dans lequel l'étape de mélange de l'alcane et du brome est réalisée à un rapport molaire de l'alcane au brome compris entre 1:2 à 10:1.

17. Procédé selon l'une quelconque des revendications 1 à 5 et 7 pour convertir le méthane en éther diméthylique, dans lequel:
l'alcane est le méthane;
le bromure d'hydrogène, formé en mélangeant le méthane et le brome, est piégé en le faisant réagir avec un oxyde de métal; et
le bromure de méthyle formé en mélangeant le méthane et le brome est dirigé dans un engagement avec un oxyde de métal, formant ainsi l'éther diméthylique et un bromure de métal.

18. Procédé selon la revendication 17, dans lequel l'étape de mélange du méthane et du brome est réalisée à un rapport molaire du méthane au brome compris entre 1:5 et 50:1.

19. Procédé selon la revendication 17, dans lequel l'étape de mélange du méthane et du brome est réalisée à un rapport molaire du méthane au brome compris entre 1:2 et 10:1.

20. Procédé selon la revendication 17, dans lequel l'étape de piégeage du bromure d'hydrogène par sa réaction avec un oxyde de métal produit de l'eau et un bromure de métal.

21. Procédé selon la revendication 20, dans lequel l'oxyde de métal qui réagit avec le bromure de méthyle et l'oxyde de métal qui réagit avec le bromure d'hydrogène sont les mêmes oxydes de métal.

22. Procédé selon la revendication 20, dans lequel l'oxyde de métal qui réagit avec le bromure de méthyle et l'oxyde de métal qui réagit avec le bromure d'hydrogène sont des oxydes de métal différents.
